# EUROPEAN PATENT APPLICATION

(11) **EP 4 799 549 A1**
(43) Date of publication of application: **02.09.2026**
(21) Application number: 26160396.3
(22) Date of filing: 24.02.2026
(51) Int. Cl.: A61B 1/00, A61B 1/045

(54) **USER INTERFACE NAVIGATION, CONTROL, AND DISPLAY**

(30) Priority: 28.02.2025 US 202563765228 P
(71) Applicant: Karl Storz SE & Co. KG, 78532 Tuttlingen (DE)
(72) Inventor: WOCHNER, Tobias, 78532 Tuttlingen (DE); KARGE, Thorsten, 78532 Tuttlingen (DE); BAUMBERGER, Juri, Goleta, 93117 (US); RUTLAND, Timothy, Goleta, 93117 (US); NAUMU, Natalie, Goleta, 93117 (US)

(57) **Abstract**

An exemplary aspect is generally directed to generating various compositions of input video information for streaming to one or more displays. For example, a first display can be configured to show a first video stream and a second video stream. The first video stream can be a live view of video from an endoscope, and the second video stream can be from a (remote) camera control unit. These streams are presented in a PIP (Picture-in-Picture) mode, or in a PAP (Picture-and-Picture) mode. Any of the video streams or other information are selectable for display and any combination thereof is selectably configured for streaming to any selected display. Additional aspects relate to a video rendering system where the dominant image is a 3D image stream and the subordinate image (such as in PiP) is the same image stream, but in 2D. Even more aspects relate to a video rendering system where the dominant image is a 2D image stream and the subordinate image is the same image stream, but in 3D.

## Description

### TECHNICAL FIELD

The present invention refers to a video system and a display method for video streaming, particularly for video processing. More specifically, the present invention refers to a video system and a display method for video streaming related to medical imaging. Even more specifically, the present invention refers to a video system and a display method for video streaming related to a user interface that facilitates image display and customization.

### BACKGROUND

The present disclosure relates to managing and displaying medical imaging as for example provided by endoscopic devices. Particularly, the present disclosure relates to technological frameworks for user interface navigation and endoscopic video stream display, centered on improving surgeon orientation, control, and workflow efficiency. For example, graphical user interfaces (GUIs) enable intuitive control of variable direction-of-view endoscopes via selectable view vectors, as well as sensor-based visualization of the three-dimensional orientation of the endoscope's distal tip. These systems support safer navigation by informing the user of spatial orientation and, in some cases, constraining movement to prevent over-bending.

Existing systems for video display management include automatic reorientation of live video streams to maintain a consistent viewing orientation, digital panning, and virtual horizon functions enabled by multi-sensor imaging at the distal tip. These features are integrated into modular, camera-agnostic control architectures, typically centered around a camera control unit that manages image processing, user interfaces, and multiple video sources. Additional aspects include touch-based interactive displays, programmable camera head controls, magnetic input interfaces, and electromagnetic tracking systems that overlay navigation data directly onto the video display, collectively forming a mature ecosystem for endoscopic UI navigation and visualization. However, current navigation systems cannot show multiple video inputs simultaneously and/or take an external video input and combine it with a currently active imaging camera.

It is an object of the present invention to provide a video system and a display method for video streams which overcome the drawbacks of the prior art. Particularly, it is an object to enhance usability and reliability of imaging systems by improving the user interface navigation, imaging control system and the video routing capabilities.

### SUMMARY

The present disclosure is directed to a video system and a display method for video streams as defined by independent claims 1 and 11. Various embodiments and method variations for the video system and the display method are disclosed in the dependent claims.

The video system comprises one or more video inputs, a mixer, a plurality of display outputs, a user interface, and at least one display. The mixer is configured to output a number of different selected ones of the one or more video inputs that were selected in the user interface as respective video streams via ones of the plurality of video outputs to the at least one display.

The display method for video streams comprises the steps of receiving a plurality of input video streams, selecting one or more of the video streams for output to a first display, and selecting one or more of the video streams for output to a second display. At least one video stream for the second display is different than one selected video stream for the first display. Further, the display method comprises the steps of streaming and outputting the selected video streams to the first and second displays, respectively, wherein one of the one or more video streams is from an internal source and another of the one or more video streams is from an external source. At least one of the one or more video streams is modified based on a selected number of menu functions.

One aspect is generally directed to video processing.

Another aspect is directed to medical imagery video processing.

Even more specifically, one aspect is directed to methods of displaying medical imaging, such as endoscope or other medical camera imaging, combined with other information.

One exemplary aspect is directed to methods of displaying medical imaging information, such as from an endoscope, exoscope, or other medical camera imaging technology, with other information in a customizable composition. There can be a plurality of the customized compositions with each composition outputtable/streamable to a different display. Each customizable composition can optionally include a plurality of user selectable quick menu functions that allow further configuration of the streamed information.

One exemplary aspect is directed to methods of displaying medical imaging information, such as a video stream from an endoscope, exoscope, or other medical camera imaging technology, with other information, such as an additional video stream, training information, still image information, a CCU stream, CT scan information, x-ray information, MRI scan information, recorded video information, 3D video information, 2D video information, and in general any information useful for a medical procedure, and/or a replay of a portion of a currently underway procedure, in a customizable composition. There can be a plurality of the customized compositions with each customizable composition outputtable/streamable to a different display. Each customizable composition can optionally include a plurality of user selectable quick menu functions (and optionally sub-menu functions) that allow further configuration of the streamed information. Each output customizable composition can optionally uniquely include 2D as well as 3D streaming video information.

Yet another aspect is directed to displaying multiple video or other inputs simultaneously and/or taking an external input and combining that external input(s) with a currently active streaming camera input.

Other aspects are directed to mixing certain input(s) in certain compositions for different output to different monitors/displays devices.

Still further aspects are directed to taking two distinct compositions each of which can go to any of two (or more) video outputs.

Additional aspects relate to taking one external video source (such as from a different CCU (Camera control Unit), reference still, reference video, prior video, training video, archived video, the Internet, or the like) and adding an internal video source (such as from an endoscope, exoscope, etc.), and rendering the combination of images/videos as a Picture-in-Picture/Picture-and-Picture, or the like.

Other aspects allow for multiple attached display devices to display their own distinct combination of the various inputs.

Additional aspects relate to using a second video input, with preferences specified by the user/system, to generate an output showing a first video stream and a second video stream. This second video stream can be from a camera control unit, and the streams are presented in a PiP (Picture-in-Picture) mode, or in a PaP (Picture-and-Picture) mode.

Even more aspects relate to a video rendering system where the dominant image is a 3D image stream, and the subordinate image is the same image stream (or a different image stream), but in 2D. For example, the subordinate image can be rendered as PiP or PaP. The size of the dominant image and the subordinate image are user adjustable. And their position on the display is user configurable.

Even more aspects relate to a video rendering system where the dominant image is a 2D image stream, and the subordinate image is the same image stream (or a different image stream), but in 3D. For example, the subordinate image can be rendered as PiP PaP. The size of the dominant image and the subordinate image are user adjustable. And their position on the display is user configurable.

Additional aspects relate to a user interface configured to control any of the above as well as to control video routing, video preferences, configuration preferences and/or video management.

Other aspects relate to providing video effect customizations via a user interface.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material".

The term "computer-readable medium" as used herein refers to any tangible storage and/or transmission medium that participate in providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored.

The terms "determine", "calculate" and "compute," and variations thereof, as used herein, are used interchangeably and include any type of methodology, process, mathematical operation or technique.

These and other features and advantages of this technology are described in, or are apparent from, the following detailed description of the exemplary embodiments.

### BRIEF DESCRIPTION OF THE DRAWINGS

The exemplary embodiments will be described in detail, with reference to the following figures, wherein:
FIG. 1 illustrates a block diagram of an exemplary video processing system according to one aspect.
FIG. 2 illustrates an exemplary preference GUI of the video system according to one aspect.
FIG. 3 illustrates another exemplary preference GUI according to one aspect.
FIG. 4 illustrates an exemplary preference GUI according to one aspect.
FIG. 5 illustrates an exemplary display composition configuration according to one aspect.
FIG. 6 illustrates an exemplary display layout to the display compositions according to one aspect.
FIG. 7 illustrates an exemplary user interface/display layout according to one aspect.
FIG. 8 illustrates an exemplary documentation interface according to one aspect.
FIG. 9 illustrates another exemplary documentation interface according to one aspect.
FIG. 10 illustrates an exemplary preference menu according to one aspect.
FIG. 11 illustrates a method for operating the imaging system according to one aspect.
FIG. 12 illustrates an exemplary method for mixing and streaming content according to one aspect.
FIG. 13 illustrates an exemplary method for mixing and streaming content based on stored preferences according to one aspect.

### DETAILED DESCRIPTION

The exemplary embodiments of the video system and the display methof will be described in relation to video processing, video mixing, medical imaging and a corresponding user interface(s).

For purposes of explanation, numerous details are set forth in order to provide a thorough understanding of the present technological solution. It should be appreciated however that the presently disclosed technology may be practiced in a variety of ways beyond the specific details set forth herein. Furthermore, while the exemplary embodiments illustrated herein show various components of this system collocated, it is to be appreciated that the various components of the system can be located at distant portions of a distributed or local network or circuit.

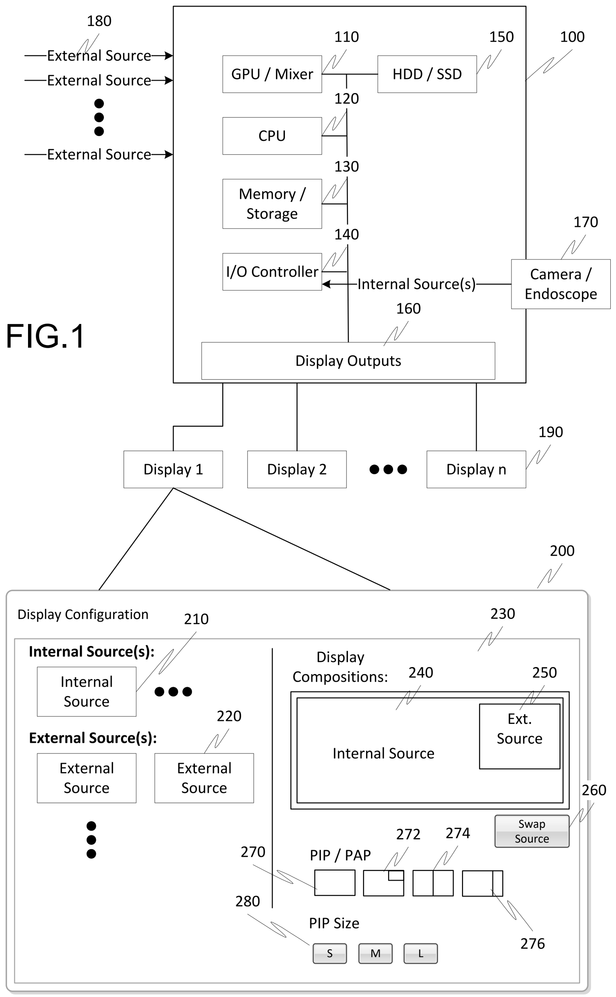

FIG. 1 illustrates a high-level block diagram of the exemplary system components. The exemplary system 100 includes a GPU/Mixer 110, a CPU 120, memory/storage 130, I/O controller 140, a hard disc drive(s)/solid state drive(s) 150, display outputs 160, one or more cameras/endoscopes 170, one or more external sources 180, and one or more displays 190.

Each of the displays 190 are configured to display a user interface with the various elements discussed herein including but not limited to video information, image information, medical information, configuration information and 2D/3D imaging, and the like.

In operation, the system receives source information from one or more sources, such as internal sources 170 (e.g., a camera/endoscope(s)/exoscope(s)) and external sources 180, such as an external video feed, image information, training videos, prior stored videos/images, internet content, information from another CCU, or any information from any source.

As will be discussed herein, the various source information is configurable for display on the one or more displays 190. Each display 190 can have the same information displayed, or a customizable mix (composition) can be selected such that one or more of the displays has a unique combination of information displayed. Of particular interest is the ability to mix 2D and 3D content on the same display and choose how the 2D and the 3D information (or other information) is displayed on the screen.

Software stored on the memory/storage 130 is responsible, with the cooperation of the CPU 120 and GPU/mixer 110 to process and display the information from the various sources based on user selected preferences. This information is then output via the display outputs 160 to the one or more displays 190, each of which can be a video display(s), a headset, a wall display, a remote display, a VR or augmented reality display, a projector, or the like, with the displayed information also capable of being stored on the hard disc drive(s)/solid state drive(s) 150, and/or stored remotely, such as on a network/server or in the cloud. The display outputs can be any known or later developed display output such as VGA, DVI-D, DVI-I, mini-DVI, micro-DVI, HDMI, mini HDMI, micro HDMI, display port (DP), Thunderbolt^{®}, USB, wireless video, SDI (Serial Digital Interface), or the like.

A user selects one or more of the sources of information for display on a display 190 in interface 200. The user (or each user at each display) can select one or more of one or more internal sources 210 and one or more external sources 220. In the display compositions section 230 of the interface 200, the user can select, for example, if the sources are to be displayed singularly 270, in a PiP or a PaP format (272/274), side-by-side in a variably adjustable format 276, one above the other, or the like. In the variably adjustable format 276, a slider can be provided that allows a user to select the slider and increase/decrease the size of one source and correspondingly decrease/increase the size of the other source. The user can also select the size of the PiP image with the PiP size selection buttons 280. While the various buttons and toggles shown herein are shown on a graphical user interface, it is to be appreciated that any of the user interface elements discussed herein could also be implemented as a physical button or a controller associated with a device located remotely, but in communication with the system, such as a button on an attached camera head. A swap source button 260 can also optionally be provided that swaps the positions of the sources within the interface.

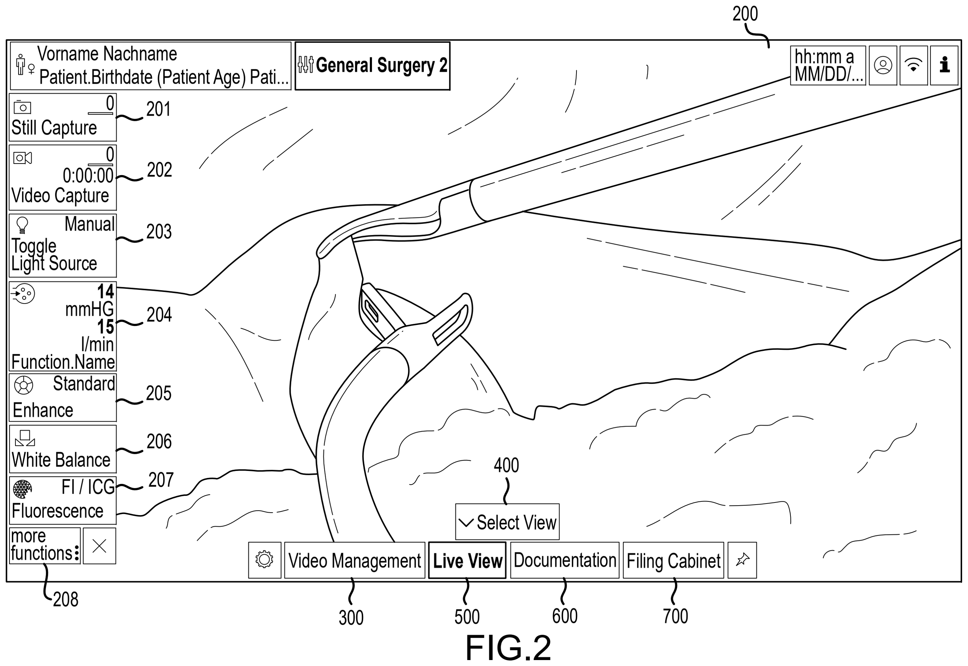

FIG. 2 shows an exemplary user interface 200 that includes, as one example, a representation of a video from an internal source, such as an endoscope, as well as a menu for selectable feature settings.

Specifically, and in addition to information such as patient information, preference information/settings, time/date information, connectivity information, help information, and the like, a menu with selectable features is provided. In this exemplary embodiment, selectable/controllable features include still capture 201, video capture 202, light source toggle 203, pressure/suction/insufflation information 204, enhance video 205, white balance 206, fluorescence 207, and more functions button 208. Each of these features may represent an installed independent feature package (IFP) which has been installed on the system 100, and by virtue of installation is now controllable within the UI.

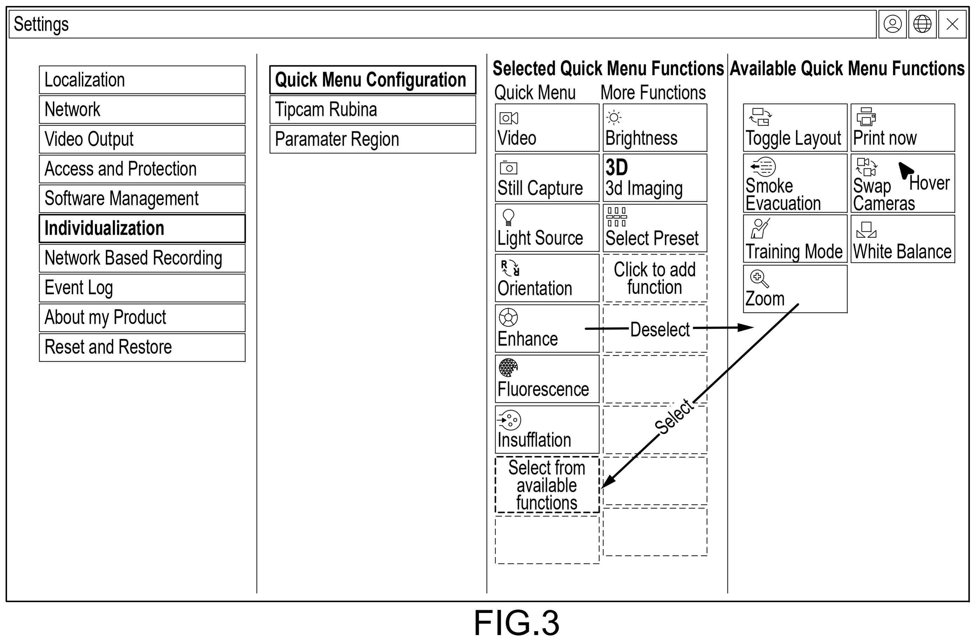

While certain features are shown, it is to be appreciated that any one or more of the features shown herein (such as those in FIGS. 3-4) can also be shown in the interface 200. Upon selection of one of these features, an optional sub-menu can be provided that allows more granular aspects of the feature to be customized/selected.

The still capture button 201 allows a user to grab a screen capture of the interface 200 or a portion thereof. Video capture button 202 allows a user to capture video of the interface 200 or a portion thereof. Light source button 203 allows a user to switch between light sources or adjust settings thereof. Insufflation button 204 provides editable pressure/volume information such as for a pump or vacuum. Image enhancement button 205 allows a user to enhance the image on interface 200 or select between a number of possible enhancement packages. White balance button 206 allows a user to change the white balance. Fluorescence button 207 controls the configuration of display of fluorescence in the image, and may include selection of a fluorescence imaging mode, display preferences (such as color overlay, sensitivity, and the like), ability to target a particular fluorophore, etc. More functions button 208 can take the user to a sub-menu(s) that allows selection/deselection of the various additional quick menu functions that can be displayed on interface 200. (See FIGS. 3-5)

Also shown in FIG. 2 are further selectable functions/buttons of the system 100 including a video management button 300, a live view button 500, a view select button 400 a documentation button 600, and a filing cabinet button 700. The video management button 300, the live view button 500, the documentation button 600, and a filing cabinet button 700 can optionally be hidden until the select view button 400 is selected.

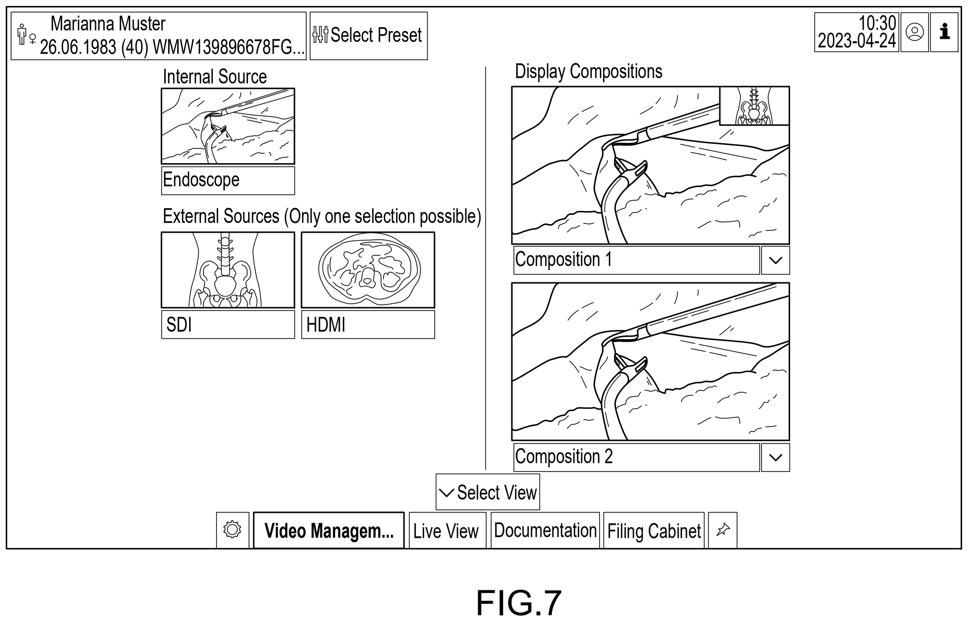

The video management button 300 allows a user to select video or other information sources and configure how those video/information sources are displayed on the interface(s) 200. As shown in FIG. 7, the left-hand side of the interface provides exemplary selectable internal video information sources (internal from the endoscope, (or other connected camera system, such as an exoscope), and external sources from SDI or an HDMI source, or the like) and the right-hand side of the interface shows exemplary display compositions for specific monitors. The upper right image in FIG. 7 is shown as a PiP with a still image present in the PiP portion. This image ("Composition 1") could be streamed to a first display. While, as an example, the lower portion ("Composition 2") provides just the live image that can be presented to another display. Any number of these compositions can be created, and the output of these compositions directed and streamed to any selected display(s).

The live view button 500 can provide the view shown in FIG. 2. And the select view button 400 could be used to, for example, display or hide various features in the interface 200.

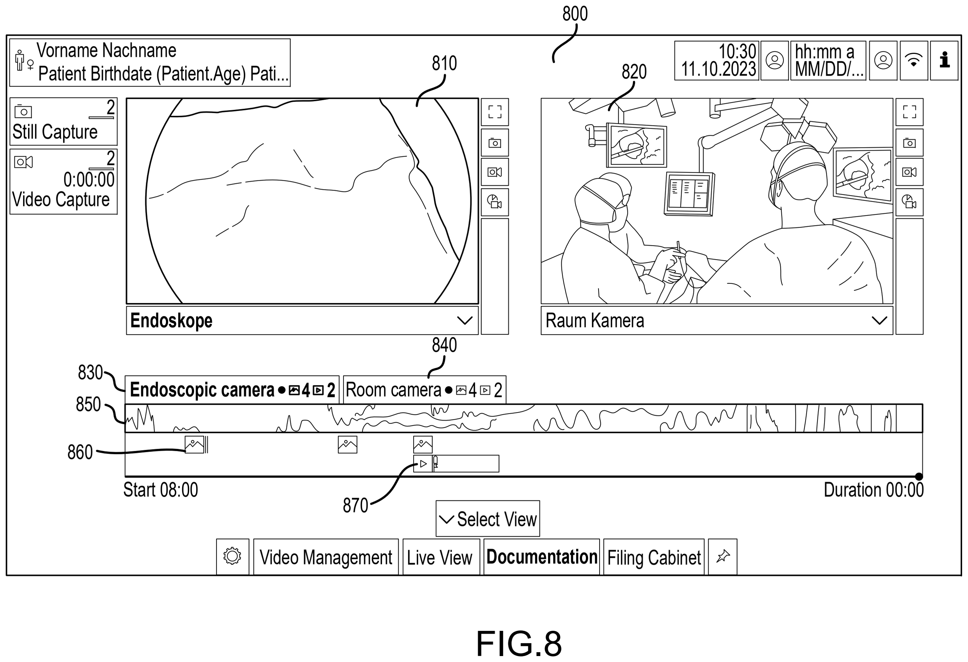

Documentation button 600 provides access to an interface that shows, for example, recorded information such as that shown in FIG. 8. Access to the "documentation" feature can be part of an optional feature package/module that can be installed on the system.

This recorded information can be stored on and accessed via the HDD/SSD 150, or can be stored remotely, such as in the cloud. In FIG. 8, the interface 800 includes an endoscope view 810 and a room view 820. As any source could have been selected as discussed, the information shown in these interfaces is customizable to any combination of input source information. FIG. 8 includes a timeline 850 for each of the endoscope camera 830 as well as the room camera 840. Using a slider a user can scroll to any point of the recorded video. Associated with the recorded video, supplemental information can be included with the recorded video such as captured still images 860 or other recorded video clips 870, documentation, notes, oral memos, metadata or in general any information a medical practitioner may associate with the recording.

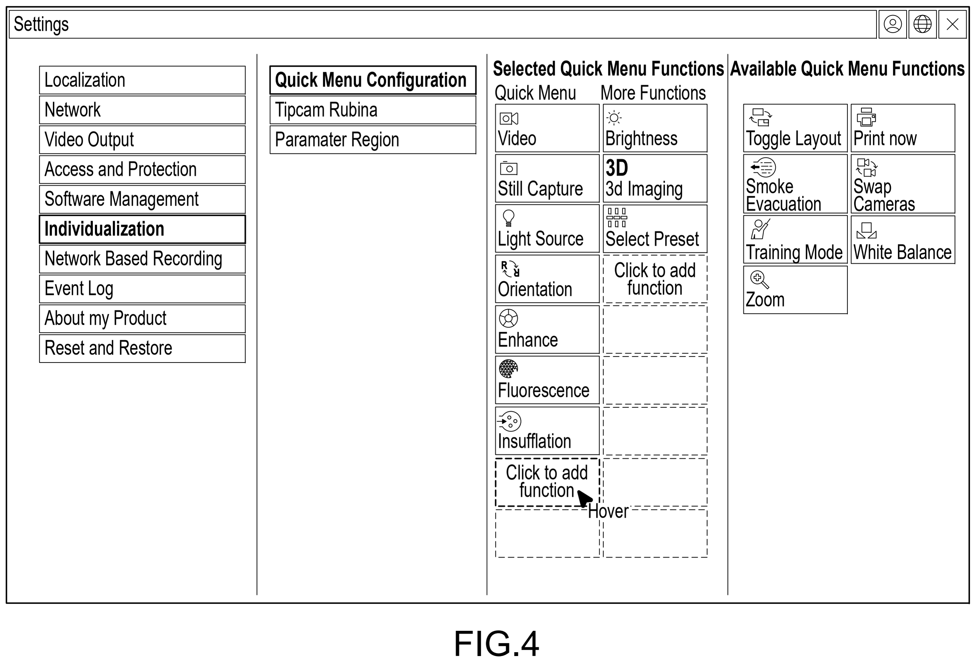

FIG. 3 shows exemplary selected quick menu functions and available quick menu functions. A user can, for example, drag and drop any of the available quick menu functions to the selected quick menu function area, and similarly deselect a quick menu function by dragging it (for example with a mouse or other input device) from the "selected" to the "available" portion of the interface. Similarly, in FIG. 4, illustrates a user selecting a blank quick menu function box to add an available quick function. For example, the user can click on an available space in the quick menu function area, thereby selecting the available space, and then click on one of the available quick menu functions to have that available quick function placed in the selected available space.

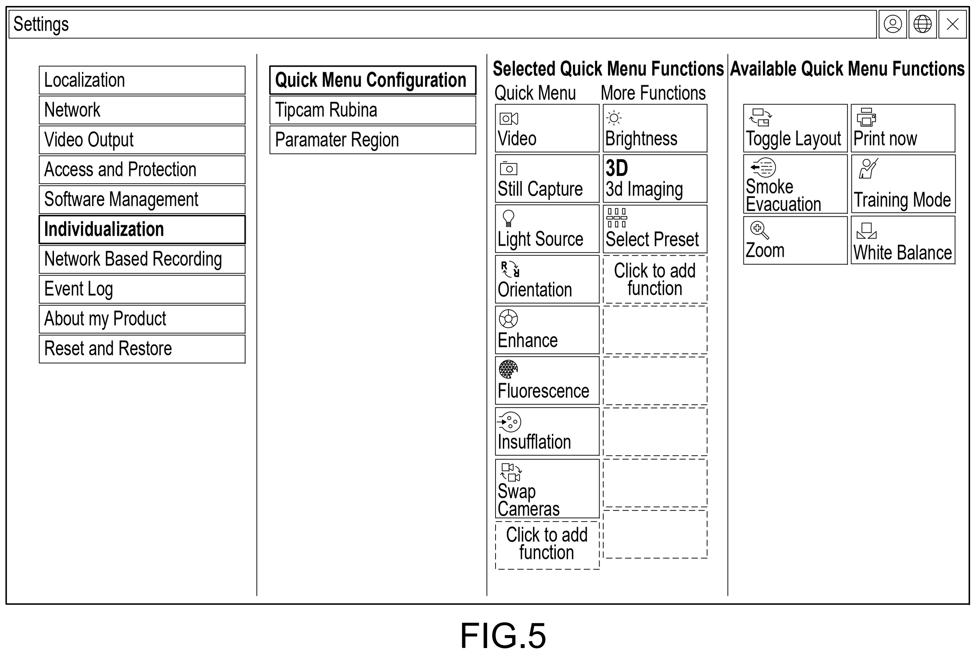

Here, the "Quick Menu" functions are those shown in the menu in FIG. 2 on the left-hand side of the interface, with the "More Functions" being accessed by selection of the "More Functions" button 208, and "Available Quick Functions" capable of being added to either category. FIG. 5 shows an exemplary menu configuration where some available quick menu functions have been added to the "Quick Menu" with these "Quick Menu" functions being displayed in a similar manner to that shown in FIG. 2. FIG. 5 also shows a software management button that allows a user to see and manage installed and available software packages, for example, on the cloud.

Some of the functions populatable and usable from the quick menu include, but are not limited to, video capture, still capture, light source selection, orientation, enhancements (video), fluorescence, insufflation, swap cameras, brightness, 3D imaging, select preset, toggle layout, smoke evacuation, white balance, print, training mode, zoom, and the like. Other quick menu functions are available, for example, based on the type of attached instruments/devices that are appropriate to those instruments/devices.

One or more of the quick menu functions can have an associated sub-menu that allows further drilling-down into the selected function. For example, selecting light source can bring up a sub-menu allowing a user to select from a plurality of illumination sources or modes. Similarly, if a user selects zoom, the sub-menu could optionally provide fixed zoom levels and/or allow the user to select a custom zoom level.

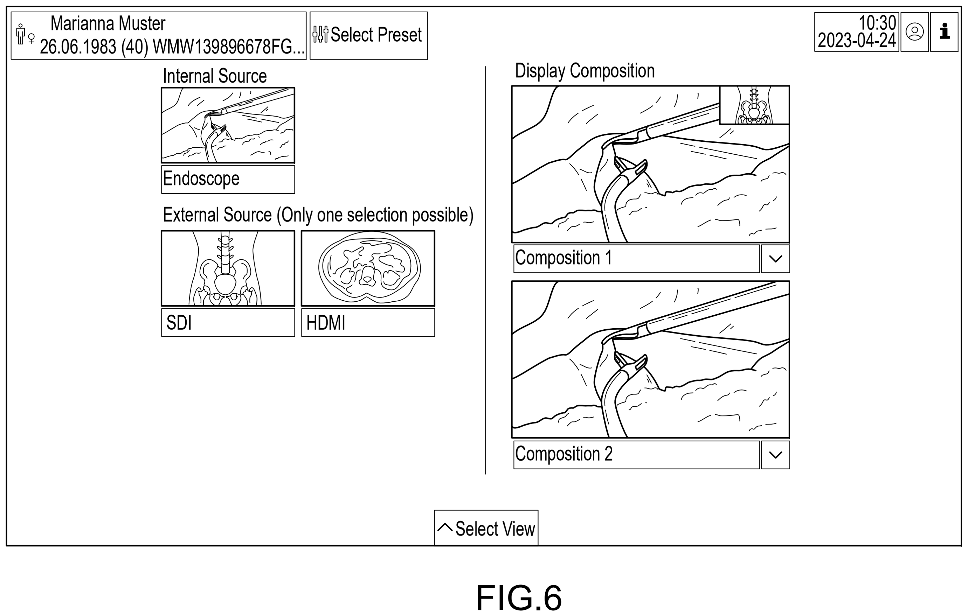

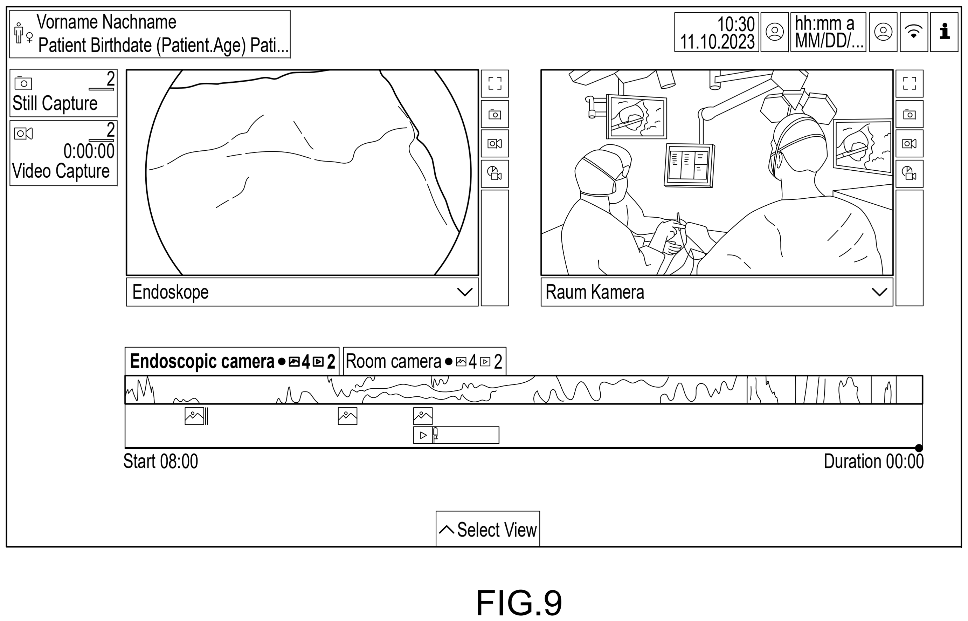

Upon selection of the select view button 400 in FIG. 2, the user can be provided with four exemplary selections: a video management button 300, a live view button 500, a documentation button 600 and a filing cabinet button 700. The live view button can display an interface such as that in FIG. 2. Upon selection of the video management button 300, an interface such as that shown in FIG. 6 can be provided that allows the user to configure the various video feeds including how they are to be displayed and where they are to be displayed. The documentation button takes a user to the exemplary interface shown in FIGS. 8-9 for accessing recorded information, such as the recording of a surgery. A surgery can be continuously recorded, and the recording of the surgery can be optionally accessed after the fact. Additionally, a user can jump anywhere in the timeline (or to any frame in the timeline) of the recording even when the surgical procedure is ongoing. This can be especially helpful to assist the medical personnel in, for example, recalling and reviewing something that has perhaps occurred earlier in a procedure.

The filing cabinet button 700 can take the user to an interface where stored information associated with the patient or historical procedures are presented. Similar to access for the "documentation" feature, the filing cabinet button and functionality can be part of an optional feature package/module that can be installed on the system 100, with that information stored locally and/or remotely.

Upon selection of the video management button 400 in FIG. 2 the user can be presented with the interface shown in FIGS. 6-7. In this video management interface, the user can select from any one or more of the internal and external sources and have them displayed on the composition side of the display. As discussed, these compositions can be configured as PiP, PaP, 2D, 3D, or some combination thereof, etc. This display composition can also be saved as a preference and recalled at a later time such that the layout of information is presented in the same manner. One particularly unique aspect is the optional ability to display 2D and 3D information together in a PiP or PaP, or side-by-side.

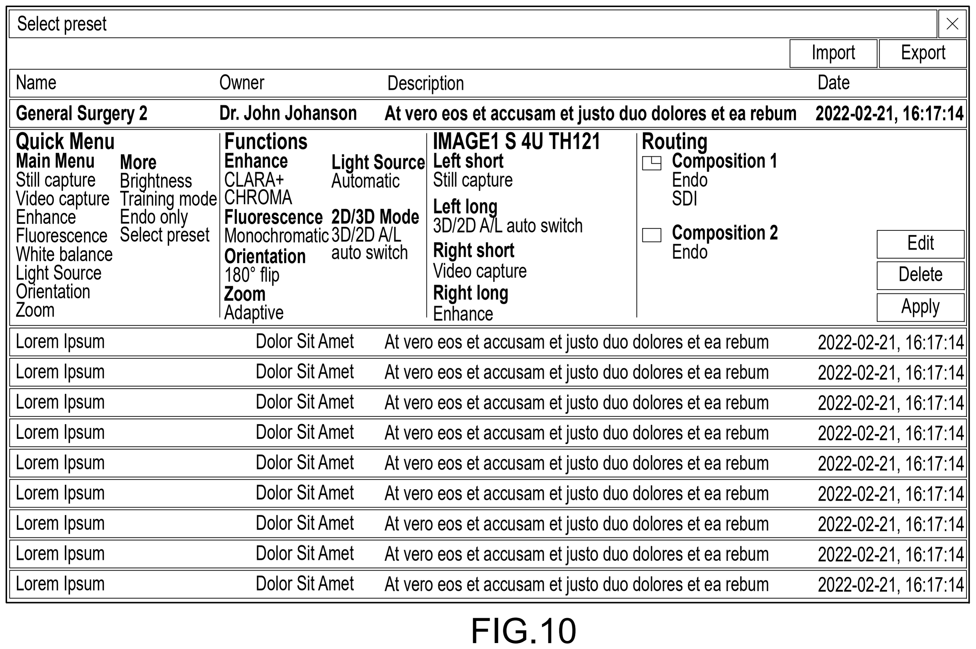

FIG. 10 illustrates an exemplary saved preference menu, "Select Preset". Here, a saved set of preferences for a specific surgical suite, and/or for a specific surgeon are selectable by selection of the selectable preset button (shown as the "General Surgery 2" button in FIG. 2, where the General Surgery 2 preset has been selected). This set of exemplary preferences has been configured to store one or more of: the quick menu main menu functions, the quick menu "more functions" menu, an order for the various quick menu functions, various presets for the selected functions (e.g., enhance, orientation, zoom, etc.), how the buttons on a medical device, such as an endoscope or camerahead, are configured, (e.g., long press for zoom, right-short press to record, etc.) and video routing information, here the input(s) for Composition 1 and the input(s) for Composition 2 are preconfigured. Specifically, exemplary Composition 1 is a PiP with video information from an endoscope and the SDI. Composition 2 displays information from the endoscope.

As will be appreciated, any feature/setup/configuration can be stored and later recalled using the save/select preset menu. These presets are also editable, deleteable and applicable with the selection of the appropriate button in the user interface. Any number of presets can be saved, and these presets can be stored on a network accessible device or the cloud and are accessible from a plurality of different systems via a network or wireless interface. In this manner, the preferences are capable of "following" a surgeon or other medical personnel throughout a hospital(s) or medical facility(ies) and are able to be quickly recalled to reduce device setup times.

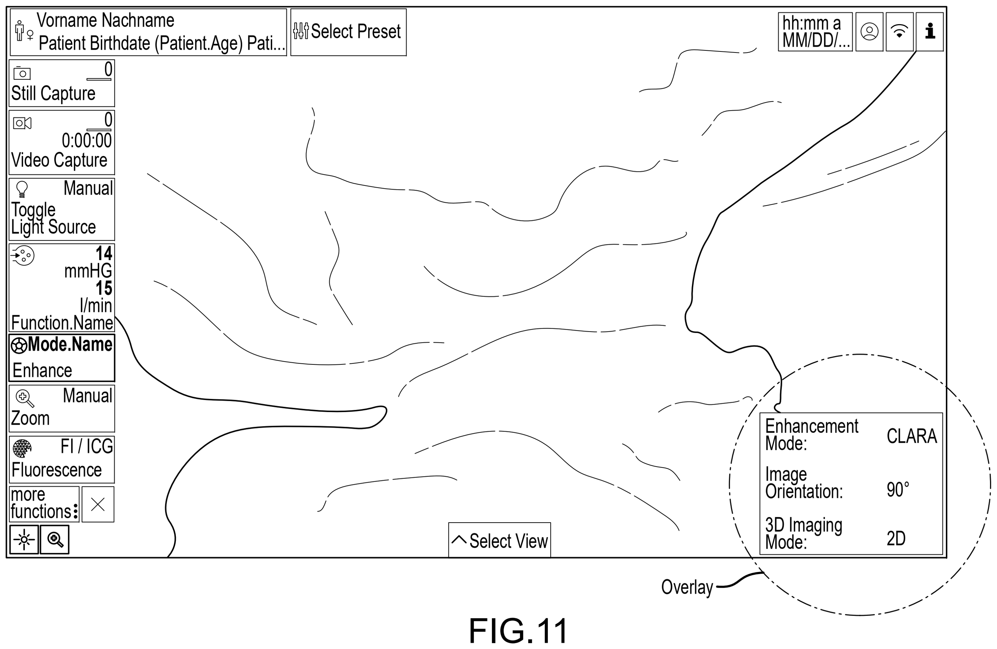

FIG. 11 also shows an optional parameter region overlay that can be included. This overlay can show certain attributes of the endoscope, e.g., enhancement mode, image orientation, 3D imaging mode, fluorescence mode, light source information, insufflation flow, insufflation pressure, light source intensity, etc. Any or all of this information can be stored in the documentation recording of the surgical procedure.

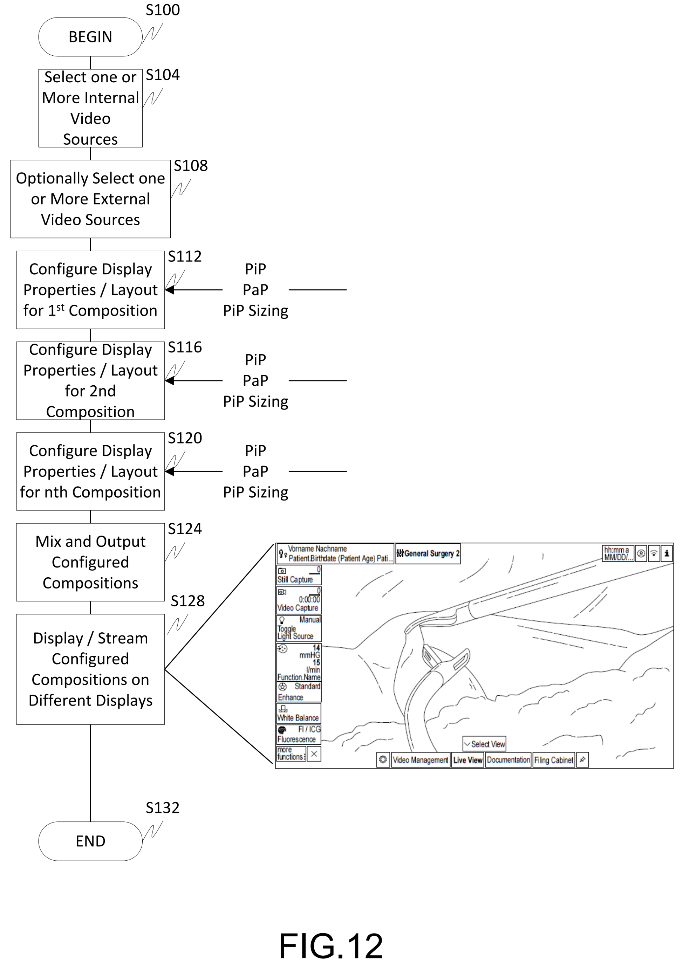

FIG. 12 outlines one exemplary method for configuring and displaying information. Control begins in step S100 and continues to step S104. In step S104, one or more internal video sources are selected. This internal video source can be, for example, from an endoscope, exoscope, or the like. Next, in step S108, one or more external sources are selected. This external source can be, for example, an external video stream, a training video, an image(s), a prior recording of a procedure, internet content, a prior scan such as a CT or MRI scan, ultrasound imaging, or in general any stored or streaming content. Control then continues to S112.

In step S112, the display properties and layout for a first composition are configured. This can include arranging the sources as PiP, PaP, selecting PiP sizing, selecting positioning and sizing, selecting where to output/stream the composition, selecting one or more display properties, and the like.

This same configuration can occur for any number of compositions as shown in S116 and S120 until all compositions are configured. Once all compositions are configured, in step S124 a mixer/GPU mixes and prepares the one or more compositions for output. Then, in step S128, the compositions are streamed to one or more displays. Control then continues to step S132 where the control sequence ends.

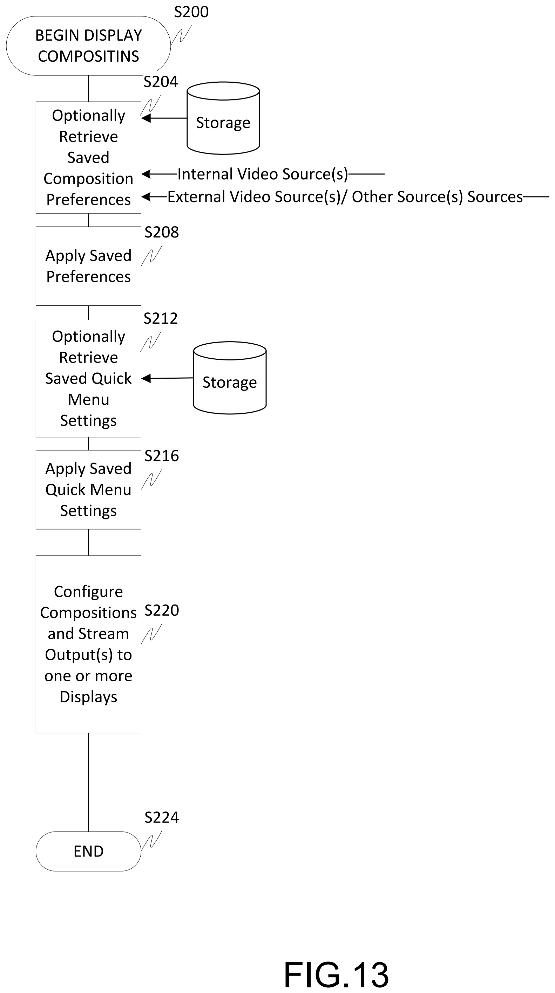

FIG. 13 outlines an exemplary method of displaying content. Control begins in step S200 and continues to step S204. In step S204, one or more saved composition preferences are retrieved. These preferences can be stored on a network accessible device, on the cloud, in the device, or in general at any accessible location. The composition preferences specify how the various content sources, such as internal video sources, external video sources, and other external information sources, are displayed. As discussed, these compositions can include PiP, PaP, full-frame, a variable or fixed side-by-side view, or the like. A mixer in step S208 reads and applies these saved preferences to the one or more information sources and prepares them for streaming to one or more displays. Control then continues to step S212.

In step S212, the system can optionally retrieve one or more saved quick menu settings. As with the saved composition settings, these preferences can be stored on a network accessible device, on the cloud, in the device, or in general at any accessible location. Next, in step S216, the saved quick menu settings are applied to the output stream. Then, the composed streams are output to one or more displays. Control then continues to step S224 where the control sequence ends.

The systems, methods and protocols of this disclosure can be implemented on a special purpose computer, a programmed microprocessor or microcontroller and peripheral integrated circuit element(s), an ASIC or other integrated circuit, a digital signal processor, a flashable device, a hard-wired electronic or logic circuit such as discrete element circuit, a programmable logic device such as PLD, PLA, FPGA, PAL, an image processing device, an endoscope, a medical imaging device, or the like. In general, any device (or one or more equivalent means) capable of implementing a state machine that is in turn capable of implementing the methodology illustrated herein can be used to implement the various methods, protocols and techniques disclosed herein.

Furthermore, the disclosed methods may be readily implemented in software stored on a non-transitory computer-readable information storage media using, for example, object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosed technology is dependent on the speed and/or efficiency requirements of the system, the particular function, and the particular software or hardware systems or microprocessor or microcomputer systems being utilized. The systems, methods and protocols illustrated herein can be readily implemented in hardware and/or software using any known or later developed systems or structures, devices and/or software by those of ordinary skill in the applicable art from the functional description provided herein and with a general basic knowledge of the computer, medical, optical and/or endoscope arts.

Moreover, the disclosed methods may be readily implemented in software that can be stored on a computer-readable storage medium, executed on programmed general-purpose computer with the cooperation of a controller and memory, a special purpose computer, a microprocessor, or the like. The systems and methods of this technology can be implemented as a program embedded on personal computer such as an applet, JAVA^{®} or CGI script, as a resource residing on a server or computer workstation, as a routine embedded in a dedicated system or system component, or the like. The system can also be implemented by physically incorporating the system and/or method into a software and/or hardware system, such as the hardware and software systems of an imaging/medical/electronic device.

While this description is described in terms of exemplary embodiments, it should be appreciated that individual aspects of the technology could be separately claimed and one or more of the features of the various embodiments can be combined.

While the exemplary embodiments illustrated herein discuss the various components collocated, it is to be appreciated that the various components of the system can be located a distant portions of a distributed network, such as a telecommunications network and/or the Internet or within a dedicated communications network. Thus, it should be appreciated that the components of the system can be combined into one or more devices or collocated on a particular node of a distributed network, such as a communications network. As will be appreciated from the following description, and for reasons of computational efficiency, the components of the system can be arranged at any location within the distributed network without affecting the operation of the system.

Although the present disclosure describes components and functions implemented in the aspects, embodiments, and/or configurations with reference to particular standards and protocols, the aspects, embodiments, and/or configurations are not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various aspects, embodiments, and/or configurations, includes components, methods, processes, systems and/or apparatus substantially as depicted and described herein, including various aspects, embodiments, configurations embodiments, sub-combinations, and/or subsets thereof. Those of skill in the art will understand how to make and use the disclosed aspects, embodiments, and/or configurations after understanding the present disclosure. The present disclosure, in various aspects, embodiments, and/or configurations, includes providing devices and processes in the absence of items not depicted and/or described herein or in various aspects, embodiments, and/or configurations hereof, including in the absence of such items as may have been used in previous devices or processes, e.g., for improving performance, achieving ease and\or reducing cost of implementation.

The foregoing discussion has been presented for purposes of illustration and description. The foregoing is not intended to limit the disclosure to the form or forms disclosed herein. In the foregoing Detailed Description for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

It is therefore apparent that there has been provided, in accordance with the present disclosure, video systems and display methods for streaming content. While this disclosure has been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the spirit and scope of the disclosed technology.

## Claims

1. Video system (100) comprising:
one or more video inputs (170, 180);
a mixer (110);
a plurality of display outputs (160);
a user interface (200); and
at least one display (190),
wherein the mixer (110) is configured to output a number of different selected ones of the one or more video inputs (170, 180) that were selected in the user interface (200) as respective video streams via ones of the plurality of video outputs (160) to the at least one display (190)

2. Video system (100) according to claim 1, wherein ones of the video inputs (170, 180) are in 2D and others of the video inputs are in 3D.

3. Video system (100) according to either of the above claims, wherein one of the one or more video inputs (170, 180) is an internal source (170) and another of the one or more video inputs is an external source (180).

4. Video system (100) according to any of the above claims, wherein the user interface (200) includes one or more buttons (270, 272, 274, 276, 260) that allow for one or more of video enhancements, picture-in-picture settings, picture-and-picture settings, and a swap source setting.

5. Video system (100) according to any of the above claims, wherein the output of the mixer (110) is recorded in real-time by storage (150).

6. Video system (100) according to any of the above claims, wherein the user interface (200) provides a button (300) to select reviewing of a prior portion of a medical procedure that is currently being performed and recorded.

7. Video system (100) according to any of the above claims, wherein a menu and a sub-menu are provided in the user interface (200), the menu providing selectable options for a plurality of functions, and the sub-menu providing additional selectable options for a selected function of the plurality of functions.

8. Video system (100) according to any of the above claims, wherein the user interface (200) is configured to allow a user to select ones of the one or more video inputs (170, 180) for specific compositions.

9. Video system (100) of the preceding claim, wherein the selected ones include 2D and 3D video streams.

10. Video system (100) according to any of the above claims, further comprising a selectable preset button (700) configured to recall one or more mixer and preference settings.

11. Display method for video streams comprising:
receiving a plurality of input video streams (170, 180), in particular video streams of a medical procedure or related to a medical procedure;
selecting one or more of the video streams for output to a first display (190);
selecting one or more of the video streams for output to a second display (190),
wherein at least one video stream for the second display (190) is different than one selected video stream for the first display (190); and
streaming and outputting the selected video streams to the first and second displays (190), respectively,
wherein one of the one or more video streams is from an internal source (170) and another of the one or more video streams is from an external source (180),
and at least one of the one or more video streams is modified based on a selected number of menu functions.

12. Display method according to the previous claim, further comprising continuously recording the output video streams using storage (150).

13. Display method according to any of claims 11-12, wherein the internal source is from an endoscope or an exoscope (170).

14. Display method according to any of claims 11-13, wherein a first of the one or more video streams is 2D and another is 3D, and the first 2D video stream is presented simultaneously with the 3D video stream.

15. Display method according to any of claims 11-14, further comprising providing an interface (200) with buttons (201-208) to select/deselect one or more of the menu functions.
